# EUROPEAN PATENT SPECIFICATION

(11) **EP 1 863 872 B1**
(45) Date of publication and mention of the grant of the patent: **29.10.2008**
(21) Application number: 05818137.1
(22) Date of filing: 22.11.2005
(51) Int. Cl.: C08K 5/3435, C07D 211/58

(54) **NOVEL POLYMERIC HINDERED AMINE LIGHT STABILIZERS BASED ON END FUNCTIONALIZED POLYOLEFINS AND A PROCESS FOR THE PREPARATION THEREOF.**
NEUE POLYMERE LICHTSCHUTZMITTEL VOM HALS-TYP AUF BASIS VON ENDFUNKTIONALISIERTEN POLYOELFINEN UND HERSTELLUNGSVERFAHREN DAFÜR
NOUVEAUX PHOTOSTABILISANTS DE TYPE AMINE A EMPECHEMENT STERIQUE POLYMERE BASES SUR DES POLYOLEFINES D' EXTREMITES FONCTIONNALISEES ET PROCEDE POUR LEUR PREPARATION

(30) Priority: 31.03.2005 IN DE07972005
(43) Date of publication of application: 12.12.2007
(73) Proprietor: COUNCIL OF SCIENTIFIC AND INDUSTRIAL RESEARCH, Niscaire Building, 3rd Floor 14, Satsang, Vihar Marg Special Institutional Area 110 067 New Delhi (IN)
(72) Inventor: KUMMETHA, Raghunatha Reddy NATIONAL CHEMICAL LAB., Pune 411008 (IN); SINGH, Raj Pal NATIONAL CHEMICAL LAB., Pune 411008 (IN)
(74) Representative: Gordon, Kirsteen Helen
(86) International application number: PCT/IN2005/000373
(87) International publication number: WO 2006/103692

(56) References cited:
- EP-A- 0 224 720
- PAN J-Q ET AL: "SYNTHESIS AND PROPERTIES OF NEW COPOLYMERS CONTAINING HINDERED AMINE" 22 August 1996 (1996-08-22), JOURNAL OF APPLIED POLYMER SCIENCE, JOHN WILEY AND SONS INC. NEW YORK, US, PAGE(S) 1405-1412 , XP000633997 ISSN: 0021-8995 the whole document
- R. MANI, R. P. SINGH, S. CHAKRAPANI, S. SIVARAN: "Synthesis, characterization and performance evaluation of hindered amine light stabilizer end functionalized poly(ethylene-alt-propene) copolymer" POLYMER, vol. 38, no. 7, 1997, pages 1739-1744, XP002374927 cited in the application
- C-E. WIL¹N, M. AUER, J. H. NÄSMAN: "Preparation of Hindered Piperidine and Its Copolymerization with Propylene over a Supported High Activity Ziegle-Natta Catalyst" JOURNAL OF POLYMER SCIENCE PART A: POLYMER CHEMISTRY, vol. 30, 1992, pages 1163-1170, XP002374928 cited in the application

## Description

### FIELD OF THE INVENTION

The present invention relates to novel polymeric hindered amine light stabilizers (PHALS) of Formula (1) based on functionalized polyolefin and a process for preparation thereof. 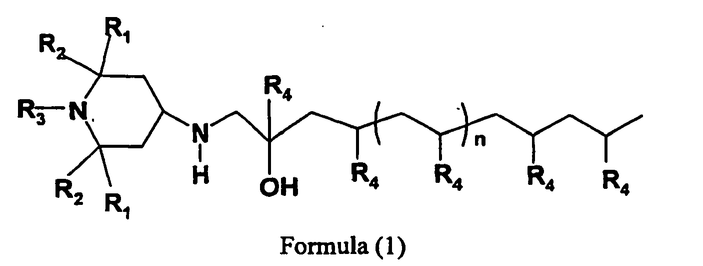 Wherein, R₁ is methyl or H, R₂ is C₁ to C₄ alkyl chain and R₃ is selected from the group consisting of H, C₁-C₈ alkyl, alkyl phenyl and alkyloxy chain and R₄ is C₁-C₈ alkyl chain. More particularly, the said PHALS of formula 1 is based on end functionalized polypropylene prepared by using of epoxy terminated polypropylene oligomers of Formula (2). 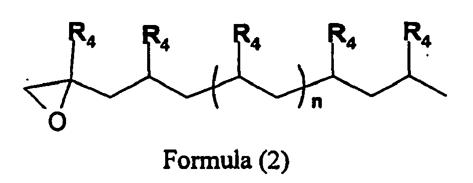 Wherein R₄ is C₁-C₈ alkyl chains, where the above epoxy end-functionalized (Formula (2)) prepared by using vinylidine-terminated polypropylene of Formula (3). 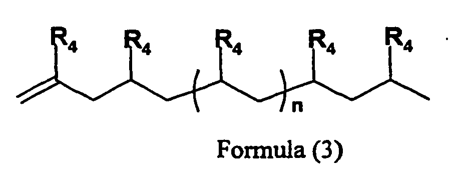 wherein R₄ is C₁-C₈ alkyl chains, where the said PHALS Formula (1) is prepared by reacting the formula 2 with 4-amino-2,2,6,6- tetramethyl piperidine containing general Formula (4). 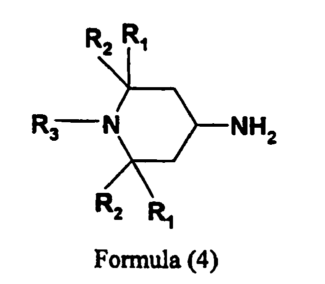 wherein R₁ is methyl or H, R₂ is C₁ to C₄ alkyl chain and R₃ is selected from the group consisting of H, C₁-C₈ alkyl, alkyl phenyl and alkyloxy chain.

### BACKGROUND OF THE INVENTION

Polymer materials exposed to sunlight or used at elevated temperatures or even stored at room temperature in presence of air undergo degradation, which shortens their service life; the former is associated with photooxidation, and the latter is mainly a consequence of thermal oxidation. These all may lead to deteriorate polymer properties like mechanical properties, impact strength and modulus etc. There are several ways to prevent thermal oxidation and photooxidation of polymers: Addition of stabilizers (antioxidants and light stabilizers) is still the most convenient and effective way as this addition will not alter processing conditions to any significant extent and there are many effective stabilizers to serve this purpose.

For outdoor use, hindered amine light stabilizers (HALS) are often added in order to prevent photodegradation of plastics, paints and textiles. The addition of polymeric HALS to polymers is used frequently due to their lower leach-out from the substrate materials. It is well known that Hindered Amine Light Stabilizers (HALS) are very effective. However, HALS with low molecular weights are relatively volatile at processing temperatures and easily extracted by liquids. A new trend in the development of light stabilizer is to prepare HALS with higher molecular weight. Copolymerization of functional monomers containing hindered amine is one method for preparing HALS with higher molecular weight. Synthesis of several polymeric HALS has been reported, many of them are based on derivatives of 2,2,6,6-tetramethyl-4-piperidene. Alkylated piperidine derivatives US pat. Nos. 4,234734 and 4,316,837 and copolymers of piperidine with olefin monomers US 5,990,209 are included as references.

Homogeneous metallocene catalyzed polyolefins have controlled molecular structure and narrow molecular weight distribution. The β-hydrogen elimination from growing chain to monomer/solvent/co-catalyst leads to form vinylidene-terminated polyolefins, functionalized via simple organic reactions to get end-functionalized polyolefins.

The use of low molecular weight (LMW) HALS compounds may not be compatible with hydrophobic polymers and also may not disperse properly because of one or more polar groups present in them. LMW compounds can migrate easily to the liquids particularly application like fiber, packaging and paints. US Patent 4,413,096 disclose the preparation of olefin copolymers containing pendent hindered amine groups. The drawbacks of polar HALS copolymers exhibit poor dispersions with hydrophobic polyolefin polymers.

Wilen et al (Journal of polymer Science Part A. Poly Chem.1992, 1163) synthesized copolymers containing hindered piperidine groups with propylene via Ziegler Natta polymerization. The disadvantage of this method was the polar groups in piperidine, which might act as poison for the catalyst and ultimately lead to give low yields of polymerization and less insertion of comonomer.

Wilen et al. (Macromolecules 2000, 33,5501) synthesized copolymers based on alkylated piperidine derivatives with ethylene and propylene. The drawbacks of the method are the comonomers based on HALS may poise the metallocene catalyst and the incorporation of comonomer will be very less.

Mani et al. (Polymer 1997, 38,1739) have prepared HALS end functionalized poly(ethylene-*alt-*propylene) copolymer by terminating anionic living polymerization of isoprene with 4-(2,3-epoxypropoxy)-1,2,2,6,6-pentamethylpiperidine followed by hydrogenation. The drawback of this method was the long route of synthesis and expensive.

Auer et al. (Journal of polymer Science Part A: Polymer Chemistry 2004, 42,1350) synthesized polymerizable hindered amine light stabilizer monomers for transition metal catalyzed olefin copolymerization with long aliphatic chain. The drawbacks of this method are the increase in feed ratio of monomer and decrease in catalyst activity.

Some preferred metallocene catalysts for the synthesis of vinylidine terminated polyolefins are bis(cyclopentadienyl) zirconiumdichloride, dimethyl bis(cyclopentadienyl)zirconium dichloride, bis(methylcyclopentadienyl)zirconium chloride and dimethylsilyl dicyclopentadienyl chloride. The co-catalysts, which may be used, are alumoxanes, which may be in the form of oligomers or cyclic alumoxanes. The alumoxanes may be prepared by contacting water with a solution of aluminum trialkyls in suitable organic solvents. The mole ratio of aluminum in an alumoxane to total metal content in the catalysts may be employed in the range of 20:1 to 5000:1
used in the preparation of the catalyst system are inert hydrocarbons. The solvent should be inert to the catalyst system, such solvents are hexane, octane, toluene, xylene and cyclohaxane. The polymerizations are usually conducted at the temperatures in the range of 20-100°C and reaction time is for few hours and it is not critical. The usual pressure employed at range of 1-2000 atmosphere. After polymerization, the catalyst was deactivated by adding acidified methanol. The low molecular weight polyolefins having vinylidine unsaturation can react easily as compared to high molecular weight polyolefins having vinylidine unsaturation. The polyolefin based polymeric stabilizer of this invention may be used as stabilizer for hydrophobic polyolefins. It may be melt blended with the polymers at sufficient levels to impart stability to the polymer matrix. The quantity of the stabilizer to be used in the polymer matrix depends upon the molecular weight of the polymer and the higher molecular weight polymers will have less quantity of HALS groups and vice versa. The optimum molecular weight of the polymers used in this is in the range of 1000 to 5000. This polymeric stabilizer may be blended with polymer matrix by any known technique, which provides good mixing.

In our previous patents US 6,489,482 B2 and US 6,737,528 B2 described the novel vinylic hindered amine stabilizers and process for the preparation thereof. The limitation of these stabilizers, are need extra processing step where to anchor them on the surface of the polymer substrate. But the present stabilizers which can be melt compounded with other additives and may disperse evenly the polymer matrix due long hydrophobic polyolefin chain.

It is known that the polyalkylpiperidine radical is effective as light stabilizer for synthetic polymers. Monomeric and low molecular weight absorbers are having limitations owing to their properties of migration and leaching. This phenomenon could lead uneven distribution of UV absorbers within polymer matrix. Leaching of a light stabilizer may cause sever problem to polymer matrix that could lead to extensive photo degradation. Therefore, in order to prevent the phenomena of migration and leaching, the light stabilizers having higher molecular weight (having long hydrocarbon chain) are being developed. This class of light stabilizers would have even distribution within the polymer matrix and also they overcome the phenomena of leaching and migration.

In this invention, olefin polymers containing terminal unsaturation are characterized up to above 95% of vinylidene unsaturation at the chain ends. The terminal unsaturated polymers and the composition employed in this invention may be prepared as described in U.S. 6117962.

### OBJECTIVES OF THE INVENTION

the main object of the present invention is to provide novel end functionalized polypropylene hindered amine light stabilizers.

Yet another object is to provide novel end functionalized polypropylene hindered amine light stabilizers with high functionality of chain ends having hindered amine group.

Yet another object is to provide a class of PHALS which may be compatible with polyolefins, polystyrene, elastomers and more particularly in fiber applications and can be added in an additive portion to obtain desired photo-stability of various other polymers also.

Yet another object is to provide a process for the preparation of novel end functionalized polypropylene hindered amine light stabilizers with high functionality of chain ends having hindered amine group.

### SUMMARY OF THE INVENTION

The present invention provides novel polymeric hindered amine light stabilizers of Formula (1), 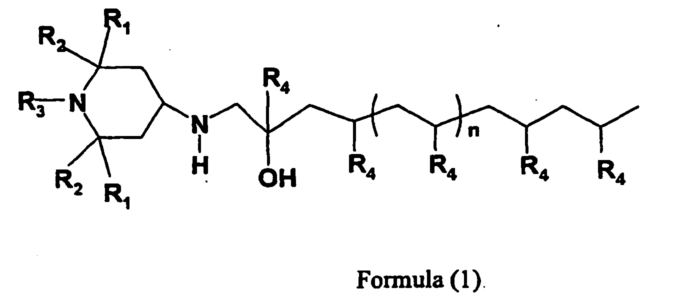 Wherein R₁ is methyl or H, R₂ is C₁ to C₄ alkyl chain and R₃ is selected from the group consisting of H, C₁-C₈ alkyl, alkyl phenyl and alkyloxy chain and R₄ is C₁-C₈ alkyl chain.

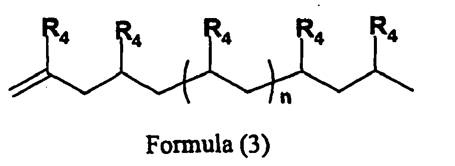

Compound of formula (3) is epoxide to obtain a compound of formula (2); 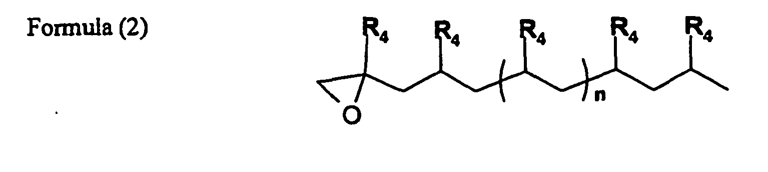 wherein R₄ is C₁-C₈ alkyl chain, subsequently the compound of formula (2) is reacted with a compound of formula (4) in an organic solvent, at a temperature in the range of 70-120 °C, at an ambient pressure, for a period of 24-36 hrs followed by washing the resultant mixture with water and drying at 30-60 °C temperature under vacuum to obtain compound of formula (1).

### DETAIL DESCRIPTION OF THE INVENTION

Accordingly the present invention provides a novel poly hindered amine light stabilizer having the formula 1 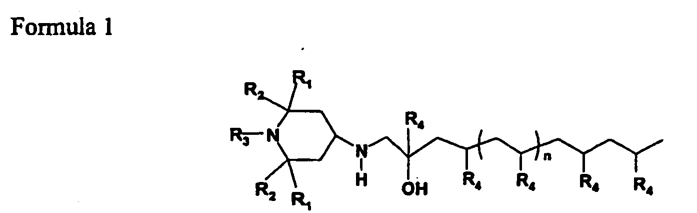 wherein R₁ is methyl or hydrogen, R₂ is C₁ to C₄ alkyl chain and R₃ is selected from the group consisting of H, C₁-C₈ alkyl, alkyl phenyl and alkyloxy chain and R₄ is C₁ to C₈ alkyl chain.

In an embodiment of the present invention the poly hindered amine light stabilizer obtained is selected from 4-amino -2,2,6,6-tetramethyl piperidine end functionalized polypropylene and 4-amino -2,2,6,6-tetramethyl piperidine end functionalized poly (hexene1)

In yet another embodiment the poly hindered amine light stabilizer obtained is selected from N-(2-hydroxypoly (propenyl)-(2,2,6,6-tetramethylpiperidinyl) amine having Mw1500 and N-(2-hydroxypoly(propenyl)-(2,2,6,6-tetramethyl pip eridinyl) amine having Mw 2200.

The present invention further provides a process for the preparation of polymeric hindered amine light stabilizer (PHALS) having the formula 1 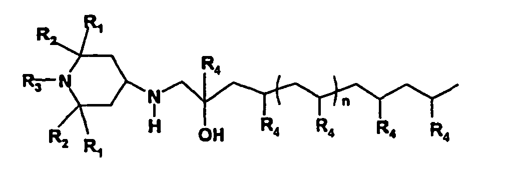 wherein, and R₁ is methyl or hydrogen, R₂ is C₁ to C₄ alkyl chain and R₃ is selected from the group consisting of H, C₁-C₈ alkyl, alkyl phenyl and alkyloxy chain and R₄ is C₁ to C₈ alkyl chain which comprises
reacting m-chloroperbenzoic acid with a compound of formula (3) 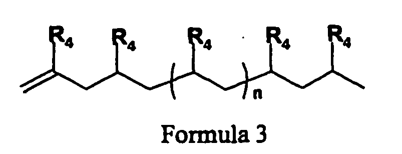 wherein R₄ is C₁-C₈ alkyl chain, in an organic solvent, at a temperature in the range of 20-40 °C, for a period of 16-24 hrs, neutralizing the resultant mixture with a saturated weak alkali sodium bicarbonate solution followed by purification and drying under vacuum by known method to obtain the compound of formula 2 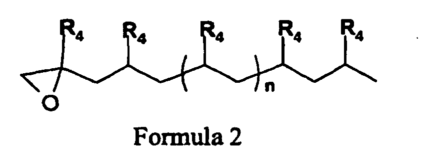 wherein R₄ is C₁-C₈ alkyl chain, reacting the above said compound of formula 2 with a hindered amine light stabilizer of formula 4 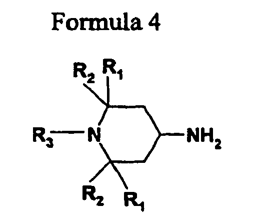 wherein R₁ is methyl or H, R₂ is C₁ to C₄ alkyl chain and R₃ is selected from the group consisting of H, C₁-C₈ alkyl, alkyl phenyl and alkyloxy chain, in an organic solvent, at a temperature in the range of 60-120 °C, for a period of 24-36 hrs, washing the resultant mixture with water and drying at a temperature of 30-60 °C under vacuum to obtain the desired compound of formula (1).

In yet another embodiment the compound of formula 3 used is having at least 10 % terminal unsaturation and is selected from crystalline and amorphous homopolymers having Mn in the range of 500 to 20,000 and crystalline and amorphous copolymers of C₂ to C₈ mono olefins having M*ₙ* in the range of 500 to 20,000.

In yet another embodiment the compound of formula 3 used is selected from terminally unsaturated poly (propylene), terminally unsaturated poly (hexenel) and copolymer of ethylene and polypropylene.

In yet another embodiment the compound of formula 3 used is selected from terminally unsaturated poly (propylene) having the number average molecular weight (Mn) of 1500 and unsaturated poly (propylene) having the number average molecular weight (Mn) of 2200.

In yet another embodiment the compound of formula 2 used is selected from epoxy end functionalized crystalline and amorphous homopolymers having Mn in the range of 500 to 20,000 and epoxy end functionalized crystalline and amorphous copolymers of C₂ to C₈ mono olefins having M*ₙ* in the range of 500 to 20,000.

In yet another embodiment the compound of formula 2 used is selected from epoxy end functionalized polypropylene and epoxy end functionalized poly (hexenel) and epoxy end functionalized copolymer of ethylene and polypropylene.

In yet another embodiment the compound of formula 2 used is selected from epoxy end Functionalized polypropylene having Mn 1500 and epoxy end functionalized polypropylene having Mn 2200.

In yet another embodiment the organic solvent used in the conversion of the compound of formula 2 to compound of formula 3 is chloroform.

In yet another embodiment the hindered amine light stabilizer of formula 4 used is 4-amino -2,2,6,6-tetramethyl piperidine.

In yet another embodiment the organic solvent used in the reaction of the compound of formula 2 with a HALS compound of formula 4 is selected from the group consisting of hydrocarbon and halogenated hydrocarbon.

In yet another embodiment the organic solvent used in the reaction of the compound of formula 2 with a HALS compound of formula 4 is selected from the group consisting of hexane, heptane, and p-chlorobenzene.

In yet another embodiment the poly hindered amine light stabilizer obtained is selected from 4-amino -2,2,6,6-tetramethyl piperidine end functionalized polypropylene and 4-amino -2,2,6,6-tetramethyl piperidine end functionalized poly (hexenel).

In yet another embodiment the poly hindered amine light stabilizer obtained is selected fril N-(2-hydroxypoly (propenyl)-(2,2,6,6-tetramethyl piperidinyl) amine having Mw1500 and N-(2-hydroxypoly(propenyl)-(2,2,6,6-tetramethyl pip eridinyl) amine having Mw 2200.

The following examples are given by the way of illustration and should not be construed to limit the scope of the invention.

### Example 1

### Synthesis of epoxy end functionalized polypropylene (Mn = 2200)

This example illustrates, terminally unsaturated poly(propylene) used was produced by using the metallocene/alumoxane catalyst system. The number average molecular weight (Mn) of polymer is 2200 and the terminal vinylidine unsaturation of the polymer is more than 95%.

In a dry 250 ml two necked round bottom flask equipped with stirrer and nitrogen inlet, 15 gms (6.8 m mol.) of the terminally unsaturated polypropylene and 100 ml chloroform solvent to form a solution were added. To this solution 2.7 equivalents 3.16gms (18.3 m mol.) of m-chloroperbenzoic acid was added and stirred the mixture for 24 hrs at ambient temperature. The epoxidised polymer was obtained by repeated washing of the reaction mixture with sodium bicarbonate and water solution and the product was dried. The product was purified by dissolving it in hexane and dried over on an anhydrous magnesium sulphate and vacuum dried in oven at 60°C.

The product was characterized by ¹H NMR and the percentage of functionalization was calculated with the help of number average molecular weight determined by Vapor Phase Osmometry and found to be 80%.

### Example 2

### Synthesis of epoxy end functionalized polypropylene (Mn =1500)

This example illustrates terminally unsaturated poly(propylene) used was produced by using the metallocene/alumoxane catalyst system. The number average molecular weight (Mn) of polymer is 1500 and the terminal vinylidine unsaturation of the polymer is more than 95%.

In a dry 250 ml two necked round bottom flask equipped with stirrer and nitrogen inlet 6.8 m mol. of the terminally unsaturated polypropylene and 100 ml chloroform solvent to form a solution were added. To this solution 2.7 equivalents (18.3 m mol.) of m-chloroperbenzoic acid was added and stirred the mixture for 24 hrs at ambient temperature. The epoxidised polymer was obtained by repeated washing of the reaction mixture with sodium bicarbonate and water solution and the product was dried. The product was purified by dissolving it in hexane and dried over on an anhydrous magnesium sulphate and vacuum dried in a vacuum oven at 60°C.

The product was characterized by ¹H NMR and the percentage of functionalization was calculated with the help of number average molecular weight determined by Vapor Phase Osmometry and found to be 80%.

### Example 3

### Synthesis of HALS end functionalized polypropylene (Mn = 1500)

This example illustrates, end epoxy functionalized polypropylene reacted with 4-amino 2,2,6,6-teramethyl piperidine. To a 200 ml two necked round bottom flask equipped with condenser and a nitrogen inlet 10 grams (4.5 mmol.) of epoxidised polypropylene and 1.2 equivelents 1.5 grams (9 m mol) of 4-amino 2,2,6,6- teramethyl piperidine and hexane as solvent were added. The hexane (solvent) was dried over sodium wire and added. This reaction was carried out for 24hrs at 70°C. The hexane solution precipitated repeated times in methanol to get above mention hindered amine polymeric light stabilizer.

The compound was characterized by ¹H NMR and the percentage of functionalization was calculated with the help of number average molecular weight determined by Vapor Phase Osmometry and found to be around 85%.

### Example 4

### Synthesis of HALS end functionalized polypropylene (Mn = 2200)

This example illustrates, end epoxy functionalized polypropylene reacted with 4-amino 2,2,6,6-teramethyl piperidine. To a 200 ml two necked round bottom flask equipped with condenser and a nitrogen inlet 4.5 m mol. of epoxidised polypropylene and 1.2 equivelents (9 m mol) of 4-amino 2,2,6,6- teramethyl piperidine and hexane as solvent were added. The hexane (solvent) was dried over sodium wire and added. This reaction was carried out for 24 hrs at 70°C. The hexane solution precipitated repeated times in methanol to get above mention hindered amine polymeric light stabilizer.

The compound was characterized by ¹H NMR and the percentage of functionalization was calculated with the help of number average molecular weight determined by Vapor Phase Osmometry and found to be around 85%.

### The advantages of present invention are :

1. The process comprises of commonly used organic reagents and with high functionality.
2. This compound may be dispersing uniformly in the polymer matrix, when compared to other existed stabilizers because of long hydrophobic chain.

## Claims

1. A poly hindered amine light stabilizer having the formula 1 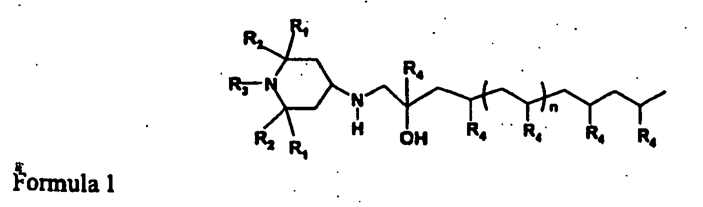 wherein R₁ is methyl or hydrogen, R₂ is C₁ to C₄ alkyl chain and R₃ is selected from the group consisting of H, C₁-C₈ alkyl, alkyl phenyl and alkyloxy chain and R₄ is C₁ to C₈ alkyl chain.

2. A poly hindered amine light stabilizer as claimed in claim 1 is selected from 4-amino - 2,2,6,6-tetramethyl piperidine end functionalized polypropylene and 4-amino -2,2,6,6-tetramethyl piperidine end functionalized poly (hexene1)

3. A poly hindered amine light stabilizer as claimed in claims 1-2 is selected from N-(2-hydroxypoly (propenyl)-(2,2,6,6-tetramethylpiperidinyl) amine having Mw1500 and N-(2-hydroxypoly(propenyl)-(2,2,6,6-tetramethyl piperidinyl) amine having Mw 2200.

4. A process for the preparation of polymeric hindered amine light stabilizer (PHALS) having the formula 1 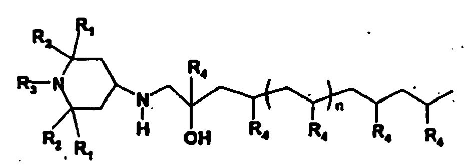 wherein, and R₁ is methyl or hydrogen, R₂ is C₁ to C₄ alkyl chain and R₃ is selected from the group consisting of H, C₁-C₈ alkyl, alkyl phenyl and alkyloxy chain and R₄ is C₁ to C₈ alkyl chain which comprises
reacting m-chloroperbenzoic acid with a compound of formula (3) 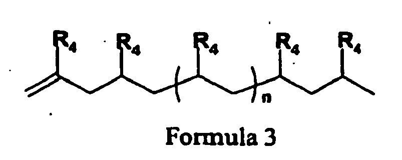 wherein R₄ is C₁-C₈ alkyl chain, in an organic solvent, at a temperature in the range of 20-40 °C, for a period of 16-24 hrs, neutralizing the resultant mixture with a saturated weak alkali 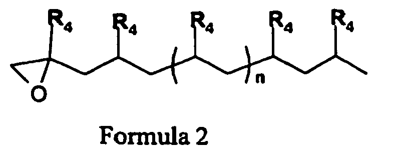 sodium bicarbonate solution followed by purification and drying under vacuum by known method to obtain the compound of formula 2
wherein R₄ is C₁-C₈ alkyl chain, reacting the above said compound of formula 2 with a hindered amine light stabilizer of formula 4 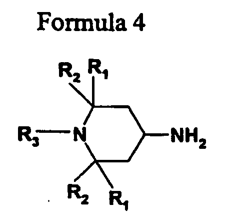 wherein R₁ is methyl or H, R₂ is C₁ to C₄ alkyl chain and R₃ is selected from the group consisting of H, C₁-C₈ alkyl, alkyl phenyl and alkyloxy chain, in an organic solvent, at a temperature in the range of 60-120 °C, for a period of 24-36 hrs, washing the resultant mixture with water and drying at a temperature of 30-60 °C under vacuum to obtain the desired compound of formula (1).

5. A process as claimed in claim 4 wherein the compound of formula 3 used is having at least 10 % terminal unsaturation and is selected from crystalline and amorphous homopolymers having Mn in the range of 500 to 20,000 and crystalline and amorphous copolymers of C₂ to C₈ mono olefins having M*ₙ* in the range of 500 to 20,000.

6. A process as claimed in claims 4-5, wherein the compound of formula 3 used is selected from terminally unsaturated poly (propylene), terminally unsaturated poly (hexenel) and copolymer of ethylene and polypropylene.

7. A process as claimed in claims 4-6, wherein the compound of formula 3 used is selected from terminally unsaturated poly (propylene) having the number average molecular weight (Mn) of 1500 and unsaturated poly (propylene) having the number average molecular weight (Mn) of 2200.

8. A process as claimed in claims 4-7, wherein the compound of formula 2 used is selected from epoxy end functionalized crystalline and amorphous homopolymers having Mn in the range of 500 to 20,000 and epoxy end functionalized crystalline and amorphous copolymers of C₂ to C₈ mono olefins having M*ₙ* in the range of 500 to 20,000.

9. A process as claimed in claims 4-8, wherein the compound of formula 2 used is selected from epoxy end functionalized polypropylene and epoxy end functionalized poly (hexenel) and epoxy end functionalized copolymer of ethylene and polypropylene.

10. A process as claimed in claims 4-9, wherein the compound of formula 2 used is selected from epoxy end functionalized polypropylene having Mn 1500 and epoxy end functionalized polypropylene having Mn 2200.

11. A process as claimed in claims 4-10, wherein the organic solvent used in the conversion of the compound of formula 3 to compound of formula 2 is chloroform.

12. A process as claimed in claims 4-11, wherein the hindered amine light stabilizer of formula 4 used is 4-amino -2,2,6,6-tetramethyl piperidine.

13. A process as claimed in claim 1, wherein the organic solvent used in the reaction of the compound of formula 2 with a HALS compound of formula 4 is selected from the group consisting of hydrocarbon and halogenated hydrocarbon.

14. A process as claimed in claims 4-13, wherein the organic solvent used in the reaction of the compound of formula 2 with a HALS compound of formula 4 is selected from the group consisting of hexane, heptane, and p-chlorobenzene.

15. A process as claimed in claims 4-14, wherein the poly hindered amine light stabilizer obtained is selected from 4-amino -2,2,6,6-tetramethyl piperidine end functionalized polypropylene and 4-amino 2,2,6,6-tetramethyl piperidine end functionalized poly (hexenel).

16. A process as claimed in claims 4-15, wherein the poly hindered amine light stabilizer obtained is selected from N-(2-hydroxypoly (propenyl)-(2,2,6,6-tetramethyl piperidinyl) amine having Mw1500 and N-(2-hydroxypoly(propenyl)-(2,2,6,6-tetramethyl piperidinyl) amine having Mw 2200.

## Patentansprüche

1. Polymerer Lichtstabilisator aus einem sterisch gehinderten Amin der Formel 1, 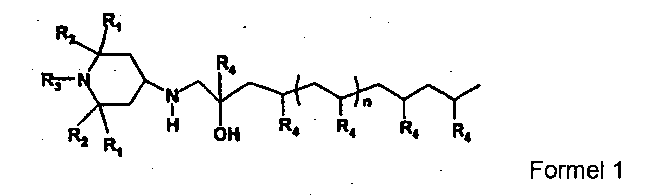 worin R₁ Methyl oder Wasserstoff ist, R₂ eine C₁ bis C₄ Alkyl-Kette ist, und R₃ ausgewählt ist aus der Gruppe bestehend aus H, einer C₁-C₈ Alkyl-, Alkylphenyl- und Alkyloxy-Kette, und R₄ eine C₁ bis C₈ Alkyl-Kette ist.

2. Polymerer Lichtstabilisator aus einem sterisch gehinderten Amin, wie in Anspruch 1 beansprucht, der ausgewählt ist aus 4-Amino-2,2,6,6-tetramethylpiperidin-endfunktionalisiertem Polypropylen und 4-Amino-2,2,6,6-tetramethylpiperidin-endfunktionalisiertem Poly(1-Hexen).

3. Polymerer Lichtstabilisator aus einem sterisch gehinderten Amin, wie in den Ansprüchen 1 - 2 beansprucht, der ausgewählt ist aus N-(2-Hydroxypoly(propenyl)-(2,2,6,6-tetramethylpiperidinyl)amin, das ein Mw von 1.500 hat, und N-(2-Hydroxypoly(propenyl)-(2,2,6,6-tetramethylpiperidinyl)amin, das ein Mw von 2.200 hat.

4. Verfahren zum Herstellen eines polymeren Lichtstabilisators aus einem sterisch gehinderten Amin (PHALS) der Formel 1 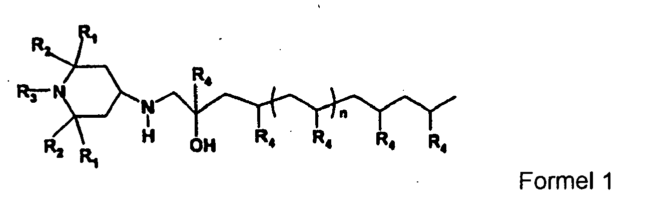 worin R₁ Methyl oder Wasserstoff ist, R₂ eine C₁ bis C₄ Alkyl-Kette ist, und R₃ ausgewählt ist aus der Gruppe bestehend aus H, einer C₁-C₈ Alkyl-, Alkylphenyl- und Alkyloxy-Kette, und R₄ eine C₁ bis C₈ Alkyl-Kette ist, das umfasst:
Umsetzen von m-Chlorperbenzoesäure mit einer Verbindung der Formel (3) 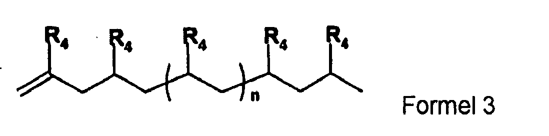
worin R₄ eine C₁-C₈ Alkyl-Kette ist, in einem organischen Lösungsmittel bei einer Temperatur im Bereich von 20-40°C, über eine Zeitdauer von 16-24 Stunden; Neutralisieren der resultierenden Mischung mit einer gesättigten, schwach alkalischen Natriumbicarbonat-Lösung gefolgt von Aufreinigung und Trocknen unter Vakuum durch eine bekannte Methode, um die Verbindung der Formel 2 zu erhalten 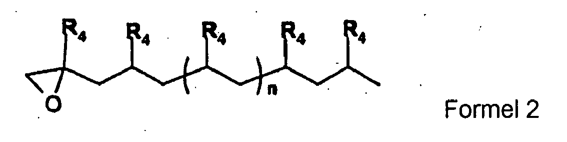 worin R₄ eine C₁-C₈ Alkyl-Kette ist;
Umsetzen der oben erwähnten Verbindung der Formel 2 mit einem Lichtstabilisator aus einem sterisch gehinderten Amin der Formel 4 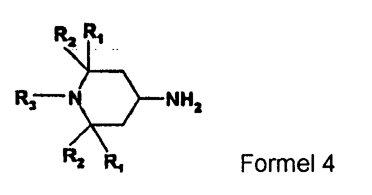 worin R₁ Methyl oder H ist, R₂ eine C₁ bis C₄ Alkyl-Kette ist, und R₃ ausgewählt ist aus der Gruppe bestehend aus H, einer C₁-C₈ Alkyl-, Alkylphenyl- und Alkyloxy-Kette, in einem organischen Lösungsmittel, bei einer Temperatur im Bereich von 60-120°C, für eine Zeitdauer von 24-36 Stunden;
Waschen der resultierenden Mischung mit Wasser und Trocknen bei einer Temperatur von 30-60°C im Vakuum, um die gewünschte Verbindung der Formel (1) zu erhalten.

5. Verfahren, wie in Anspruch 4 beansprucht, worin die verwendete Verbindung der Formel 3 mindestens 10% endständig ungesättigt ist, und die ausgewählt ist aus kristallinen und amorphen Homopolymeren, die ein Mn im Bereich von 500 bis 20.000 haben, und kristallinen und amorphen Copolymeren von C₂ bis C₈ Monoolefinen, die ein Mn im Bereich von 500 bis 20.000 haben.

6. Verfahren, wie in den Ansprüchen 4 - 5 beansprucht, worin die verwendete Verbindung der Formel 3 ausgewählt ist aus endständig ungesättigtem Polypropylen, endständig ungesättigtem Poly-1-Hexen, und Copolymer von Ethylen und Polypropylen.

7. Verfahren, wie in den Ansprüchen 4 - 6 beansprucht, worin die verwendete Verbindung der Formel 3 ausgewählt ist aus endständig ungesättigtem Polypropylen, das ein Zahlenmittel des Molekulargewichts (Mn) von 1.500 hat, und ungesättigtem Polypropylen, das ein Zahlenmittel des Molekulargewichts (Mn) von 2.200 hat.

8. Verfahren, wie in den Ansprüchen 4 - 7 beansprucht, worin die verwendete Verbindung der Formel 2 ausgewählt ist aus epoxyendfunktionalisierten kristallinen und amorphen Homopolymeren, die ein Mn im Bereich von 500 bis 20.000 haben, und epoxyendfunktionalisierten kristallinen und amorphen Copolymeren aus C₂ bis C₈ Monoolefinen, die ein Mn im Bereich von 500 bis 20.000 haben.

9. Verfahren, wie in den Ansprüchen 4 - 8 beansprucht, worin die verwendete Verbindung der Formel 2 ausgewählt ist aus epoxyendfunktionalisiertem Polypropylen und epoxyendfunktionalisiertem Poly(1-Hexen), und epoxyendfunktionalisiertem Copolymer von Ethylen und Polypropylen.

10. Verfahren, wie in den Ansprüchen 4-9 beansprucht, worin die verwendete Verbindung der Formel 2 ausgewählt ist aus epoxyendfunktionalisiertem Polypropylen, das ein Mn von 1.500 hat, und epoxyendfunktionalisiertem Polypropylen, das ein Mn von 2.200 hat.

11. Verfahren, wie in den Ansprüchen 4 -10 beansprucht, worin das organische Lösungsmittel, das bei der Umwandlung der Verbindung der Formel 3 zur Verbindung der Formel 2 verwendet wird, Chloroform ist.

12. Verfahren, wie in den Ansprüchen 4 -11 beansprucht, worin der verwendete Lichtstabilisator aus einem sterisch gehinderten Amin der Formel (4) 4-Amino-2,2,6,6-tetramethylpiperidin ist.

13. Verfahren, wie in Anspruch 1 beansprucht, worin das organische Lösungsmittel, das bei der Umsetzung der Verbindung der Formel 2 mit einer HALS-Verbindung der Formel 4 verwendet wird, ausgewählt ist aus der Gruppe bestehend aus Kohlenwasserstoff und halogeniertem Kohlenwasserstoff.

14. Verfahren, wie in den Ansprüchen 4 -13 beansprucht, worin das organische Lösungsmittel, das bei der Umsetzung der Verbindung der Formel 2 mit einer HALS-Verbindung der Formel 4 verwendet wird, ausgewählt ist aus der Gruppe bestehend aus Hexan, Heptan, und p-Chlorbenzol.

15. Verfahren, wie in den Ansprüchen 4 -14 beansprucht, worin der erhaltene polymere Lichtstabilisator aus einem sterisch gehinderten Amin ausgewählt ist aus 4-Amino-2,2,6,6-tetramethylpiperidin-endfunktionalisiertem Polypropylen und 4-Amino-2,2,6,6-tetramethylpiperidin-endfunktionalisiertem Poly-1-Hexen.

16. Verfahren, wie in den Ansprüchen 4 - 15 beansprucht, worin der erhaltene polymere Lichtstabilisator aus einem sterisch gehinderten Amin ausgewählt ist aus N-(2-Hydroxypoly(propenyl)-(2,2,6,6-tetramethylpiperidinyl)amin, das ein Mw von 1.500 hat, und N-(2-Hydroxypoly(propenyl)-(2,2,6,6-tetramethylpiperidinyl)amin, das ein Mw von 2.200 hat.

## Revendications

1. Photostabilisant de type polymère-amine encombrée de formule 1 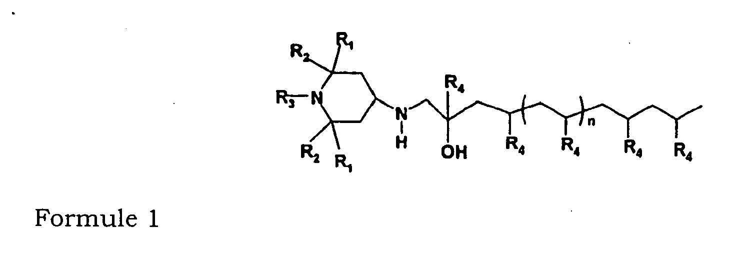 dans laquelle R₁ représente un groupe méthyle ou un atome d'hydrogène, R₂ représente une chaîne alkyle en C₁ à C₄ et R₃ est choisi dans le groupe constitué par H, les chaînes alkyle en C₁ à C₈, alkylphényle et alkyloxy et R₄ représente une chaîne alkyle en C₁ à C₈.

2. Photostabilisant de type polymère-amine encombrée selon la revendication 1, qui est choisi entre le polypropylène fonctionnalisé avec une terminaison 4-amino-2,2,6,6-tétraméthylpipéridine et le poly(hexène-1) fonctionnalisé avec une terminaison 4-amino-2,2,6,6-tétraméthylpipéridine.

3. Photostabilisant de type polymère-amine encombrée selon les revendications 1 et 2, qui est choisi entre la (N-(2-hydroxy)polypropényl)-(2,2,6,6-tétraméthylpipéridinyle)amine ayant la masse moléculaire en poids de 1 500 et la (N-(2-hydroxy)polypropényl)-(2,2,6,6-tétraméthylpipéridinyle)amine ayant la masse moléculaire en poids de 2 200.

4. Procédé de préparation d'un photostabilisant de type polymère-amine encombrée (PHALS) de formule 1 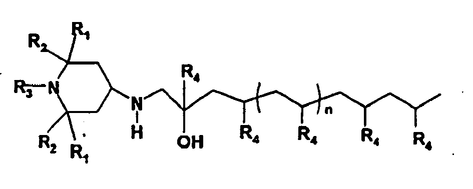 dans laquelle R₁ représente un groupe méthyle ou un atome d'hydrogène, R₂ représente une chaîne alkyle en C₁à C₄ et R₃ est choisi dans le groupe constitué par H, les chaînes alkyle en C₁ à C₈, alkylphényle et alkyloxy et R₄ représente une chaîne alkyle en C₁ à C₈
qui comprend les étapes consistant à
- faire réagir l'acide m-chloroperbenzoïque avec un composé de formule (3) 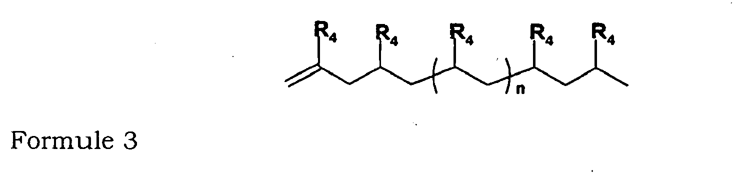
dans laquelle R₄ représente une chaîne alkyle en C₁ à C₈, dans un solvant organique, à une température dans la gamme de 20°C à 40°C, pendant une durée de 16 à 24 heures,
- à neutraliser le mélange résultant avec une solution saturée d'hydrogénocarbonate de sodium faiblement basique puis à purifier et à sécher sous vide au moyen d'un procédé connu pour obtenir le composé de formule 2 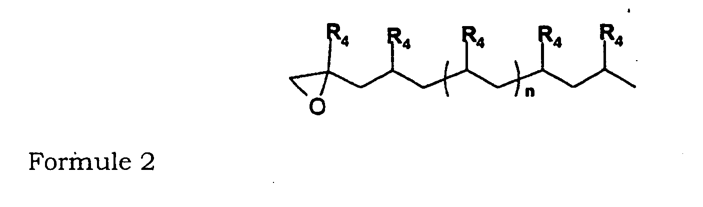
dans laquelle R₄ représente une chaîne alkyle en C₁ à C₈
- à faire réagir le composant ci-dessus de formule 2 avec le photostabilisant de type amine encombrée de formule 4 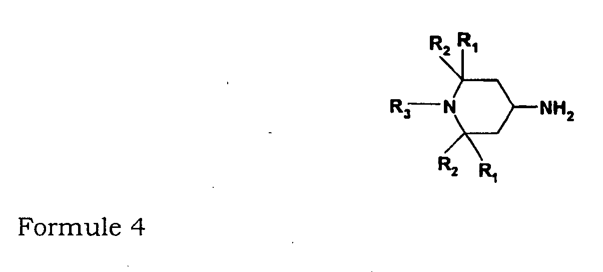
dans laquelle R₁ représente un groupe méthyle ou H, R₂ représente une chaîne alkyle en C₁ à C₄ et R₃ est choisi dans le groupe constitué par H, les chaînes alkyle en C₁ à C₈, alkylphényle et alkyloxy, dans un solvant organique, à une température dans la gamme de 60°C à 120°C, pendant une durée de 24 à 36 heures, puis à laver à l'eau le mélange résultant et à le sécher sous vide à une température de 30°C à 60°C pour obtenir le composé de formule (1) souhaité.

5. Procédé selon la revendication 4, dans lequel le composé de formule 3 utilisé comporte au moins 10% d'insaturation terminale et est choisi parmi les homopolymères cristallins et amorphes dont la Mm est dans la gamme de 500 à 20 000 et les copolymères cristallins et amorphes de monooléfines en C₂ à C₈ dont la Mn est dans la gamme de 500 à 20 000.

6. Procédé selon les revendications 4 et 5, dans lequel le composé de formule 3 utilisé est choisi parmi les polypropylènes à terminaison insaturée, les poly(hexène-1) à terminaison insaturée et les copolymères d'éthylène et de polypropylène.

7. Procédé selon les revendications 4 à 6, dans lequel le composé de formule 3 utilisé est choisi entre le poly(propylène) à terminaison insaturée ayant la masse moléculaire moyenne en nombre (Mn) de 1 500 et le polypropylène insaturé ayant la masse moléculaire moyenne en nombre (Mn) de 2 200.

8. Procédé selon les revendications 4 à 7, dans lequel le composé de formule 2 utilisé est choisi parmi les homopolymères cristallins et amorphes de monooléfines fonctionnalisés avec une terminaison époxyde dont la Mn se situe dans la gamme de 500 à 20 000 et les copolymères cristallins et amorphes de C₂ à C₈ monooléfines avec une terminaison époxyde ayant une Mn dans la gamme de 500 à 20 000.

9. Procédé selon les revendications 4 à 8, dans lequel le composé de formule 2 utilisé est choisi parmi le polypropylène fonctionnalisé avec une terminaison époxyde, le poly(hexène-1) fonctionnalisé avec une terminaison époxyde et les copolymères d'éthylène et de polypropylène fonctionnalisés avec une terminaison époxyde.

10. Procédé selon les revendications 4 à 9, dans lequel le composé de formule 2 utilisé est choisi entre le polypropylène fonctionnalisé avec une terminaison époxyde ayant une Mn de 1 500 et le polypropylène fonctionnalisé avec une terminaison époxyde ayant une Mn de 2 200.

11. Procédé selon les revendications 4 à 10, dans lequel le solvant organique utilisé dans la conversion du composé de formule 3 en composé de formule 2 est le chloroforme.

12. Procédé selon les revendications 4 à 11, dans lequel le photostabilisant de type amine encombrée de formule 4 utilisé est la 4-amino-2,2,6,6-tétraméthylpipéridine.

13. Procédé selon la revendication 1, dans lequel le solvant organique utilisé dans la réaction du composé de formule 2 avec un composé photostabilisant de type polymère-amine encombrée de formule 4 est choisi dans le groupe constitué par les hydrocarbures et les hydrocarbures halogénés.

14. Procédé selon les revendications 4 à 13, dans lequel le solvant organique utilisé dans la réaction du composé de formule 2 avec un composé HALS de formule 4 est choisi dans le groupe constitué par l'hexane, l'heptane et le p-chlorobenzène.

15. Procédé selon les revendications 4 à 14, dans lequel le photostabilisant de type polymère-amine encombrée obtenu est choisi entre le polypropylène fonctionnalisé avec une terminaison 4-amino-2,2,6,6-tétraméthylpipéridine et le poly(hexène-1) fonctionnalisé avec une terminaison 4-amino-2,2,6,6-tétraméthylpipéridine.

16. Procédé selon les revendications 4 à 15, dans lequel le photostabilisant de type polymère-amine encombrée obtenu est choisi entre la (N-(2-hydroxy)polypropényl)-(2,2,6,6-tétraméthylpipéridinyl)-amine ayant la masse moléculaire en poids de 1 500 et la N-(2-hydroxypolypropényl)-(2,2,6,6-tétraméthylpipéridinyl)amine ayant la masse moléculaire en poids de 2 200.
